(19) Europäisches Patentamt
European Patent Office
Office européen des brevets

(11) **EP 4 621 066 A1**

(12) **EUROPEAN PATENT APPLICATION**
published in accordance with Art. 153(4) EPC

(43) Date of publication:
**24.09.2025 Bulletin 2025/39**

(21) Application number: **22968093.9**

(22) Date of filing: **12.12.2022**

(51) International Patent Classification (IPC):
*C12Q 1/68* (2018.01)

(52) Cooperative Patent Classification (CPC):
**C12Q 1/68**

(86) International application number:
**PCT/CN2022/138468**

(87) International publication number:
**WO 2024/124379 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **MGI Tech Co., Ltd.**
**Shenzhen, Guangdong 518083 (CN)**

(72) Inventors:
• **ZHANG, Yuannian**
**Shenzhen, Guangdong 518083 (CN)**
• **GONG, Meihua**
**Shenzhen, Guangdong 518083 (CN)**

• **ZHANG, Yinghua**
**Shenzhen, Guangdong 518083 (CN)**
• **OUYANG, Kai**
**Shenzhen, Guangdong 518083 (CN)**
• **DING, Ya**
**Shenzhen, Guangdong 518083 (CN)**
• **XU, Chongjun**
**Shenzhen, Guangdong 518083 (CN)**

(74) Representative: **Dehns**
**10 Old Bailey**
**London EC4M 7NG (GB)**

Remarks:
The complete document including Reference
Table(s) and the Sequence Listing(s) can be
downloaded from the EPO website

(54) **MULTI-TEMPLATE NUCLEIC ACID SYNCHRONOUS SEQUENCING METHOD AND USE THEREOF**

(57) Provided are a multi-template nucleic acid synchronous sequencing method and use thereof. The method includes: providing a plurality of composite nucleic acid template spots, with a plurality of nucleic acid templates being arranged in the plurality of composite nucleic acid template spots; hybridizing the plurality of nucleic acid templates with corresponding sequencing primers thereof; performing, by use of the sequencing primers, a plurality of sequencing reaction cycles on each of the plurality of nucleic acid templates hybridized with the sequencing primers, wherein in each of the plurality of sequencing reaction cycles, signal intensities generated by the plurality of nucleic acid templates exhibit variations from one another; and classifying sequencing channel signals into the plurality of nucleic acid templates based on the variations in the signal intensities for each of the plurality of sequencing reaction cycles.

EP 4 621 066 A1

## Description

### FIELD

The present disclosure relates to the field of nucleic acid sequencing technology and biological information, and in particular to a multi-template nucleic acid synchronous sequencing method and use thereof.

### BACKGROUND

Currently, high-throughput sequencing methods mainly include single-end sequencing and paired-end/mate-paired (PE/MP) sequencing. PE/MP sequencing, also known as bi-directional sequencing, involves sequencing both ends of a long DNA fragment, producing a read "pair" whose insert size corresponds to the distance between the two end-reads, allowing for sequence assembly, alignment, and the like. For the repeats, deletions, and insertions of gene fragments, this method is more accurate and offer a wider genome coverage. The difference between paired-end and mate-paired sequencing lies in the library preparation process. Currently, paired-end sequencing has become the mainstream, as it not only extends sequencing read lengths but also provides new methods for structural variant analysis.

For paired-end sequencing, different sequencing platforms employ different strategies, but the general process involves sequencing one strand (first strand) of the DNA first, followed by sequencing its complementary strand (second strand). Many existing paired-end sequencing technologies utilize bridge amplification. This process begins with DNA fragmentation, followed by the addition of adapters to both ends. The adapters contain sequencing primer binding sites. After the first read is generated by sequencing the first strand, the first read is removed. A paired-end sequencing module generates and amplifies the complementary strand in situ. The newly synthesized complementary strands then serve as templates for the second strand sequencing.

A major drawback of current paired-end sequencing methods is that sequencing must be performed sequentially on both DNA strands, reducing sequencing throughput and increasing sequencing costs. Therefore, there is still a need to develop more flexible and efficient sequencing methods.

### SUMMARY

The present disclosure is based on the following discoveries of the inventors.

Current paired-end sequencing still requires sequential sequencing of both DNA strands, which limits sequencing throughput and results in high sequencing costs. In the present application, the inventors utilize amplification techniques to generate sequencing chips, each containing a plurality of template nucleic acid molecules, such as both the first and second strands (sense and antisense strands) of DNA, and adjuste either the relative copy numbers of these different templates within a cluster or the concentrations of their respective sequencing primers to achieve signal variations among different templates during a single sequencing reaction cycle, thereby distinguishing and positioning the bases detected from different templates. Moreover, during sequencing, crosstalk correction parameters and/or phasing correction parameters are applied to refine base-calling results. Thus, simultaneous sequencing of multiple nucleic acid templates can be achieved. This method can significantly reduce sequencing time and costs while improving sequencing throughput, making it suitable for widespread application.

Thus, in a first aspect of the present disclosure, a sequencing method is provided. According to an embodiment of the present disclosure, the sequencing method includes: providing a plurality of composite nucleic acid template spots with a plurality of nucleic acid templates being arranged in the plurality of composite nucleic acid template spots; hybridizing the plurality of nucleic acid templates with corresponding sequencing primers thereof; performing, by use of the sequencing primers, a plurality of sequencing reaction cycles on each of the plurality of nucleic acid templates hybridized with the sequencing primers, wherein in each of the plurality of sequencing reaction cycles, signal intensities generated by the plurality of nucleic acid templates exhibit variations from one another; and for each of the plurality of sequencing reaction cycles, classifying sequencing channel signals among the plurality of nucleic acid templates based on the variations in the signal intensities.

According to an embodiment of the present disclosure, the above method enables efficient simultaneous sequencing of multiple nucleic acid templates, significantly reducing sequencing time and costs while improving sequencing throughput, making it suitable for widespread application.

According to an embodiment of the present disclosure, the above sequencing method may further include at least one of the following additional technical features.

According to an embodiment of the present disclosure, the variations in the signal intensities generated by the plurality of nucleic acid templates follow a predetermined relationship. The present disclosure controls the variations in the signal intensities generated by the nucleic acid templates, and then classifies the sequencing channel signals among the plurality of nucleic acid templates. Those skilled in the art can adjust the multiplier of the the variations in the signal intensities generated by the nucleic acid templates as needed.

According to an embodiment of the present disclosure, the predetermined relationship is determined by controlling copy number variations of the plurality of nucleic acid templates or controlling concentration variations of the sequencing primers for the plurality of nucleic acid templates.

According to an embodiment of the present disclosure, controlling the copy number variations of the plurality of nucleic acid templates is achieved by controlling concentration variations of the primers for different templates or duration of polymerization extension reaction during sequencing library construction.

According to an embodiment of the present disclosure, the variations in the signal intensities generated by the plurality of nucleic acid templates are at least twofold.

According to an embodiment of the present disclosure, the plurality of nucleic acid templates are located at different positions in the same nucleic acid molecule.

According to an embodiment of the present disclosure, the plurality of nucleic acid templates are located in different nucleic acid molecules.

According to an embodiment of the present disclosure, the plurality of nucleic acid templates on the chip are located at different positions on the same single-strand DNA molecule.

According to an embodiment of the present disclosure, the plurality of nucleic acid templates on the chip are located on different strands within a complex of multiple single-stranded DNA molecules.

According to an embodiment of the present disclosure, the plurality of nucleic acid templates include a DNA molecule obtained by: forming a single-stranded DNA molecule by performing rolling circle amplification (RCA) to extend a rolling circle amplification primer, and performing multiple displacement amplification (MDA) on the single-stranded DNA molecule to extend a multiple displacement amplification primer.

According to an embodiment of the present disclosure, the rolling circle amplification primer is immobilized on a solid support or is free in a solution.

According to an embodiment of the present disclosure, the multiple displacement amplification primer is immobilized on a solid support or is free in a solution.

According to an embodiment of the present disclosure, the rolling circle amplification and the multiple displacement amplification are performed simultaneously in the same reaction system.

According to an embodiment of the present disclosure, the rolling circle amplification reaction is performed prior to the multiple displacement reaction through hybridization of the multiple displacement primer.

According to an embodiment of the present disclosure, the signal intensities generated by the plurality of nucleic acid templates in each of the plurality of composite nucleic acid template spots exhibit variations from one another. The sequencing channel signals are classified among the plurality of nucleic acid templates based on the variations in the signal intensities for each of the plurality of sequencing reaction cycles, to determine nucleotide sequences of the plurality of nucleic acid templates in each of the plurality of composite nucleic acid template spots.

According to an embodiment of the present disclosure, the plurality of nucleic acid templates include a DNA cluster obtained by PCR amplification.

According to an embodiment of the present disclosure, for each of the plurality of sequencing reaction cycles, the signals generated in each channel are subjected to intensity correction before being classified among the plurality of nucleic acid templates.

According to an embodiment of the present disclosure, the correction parameters used for intensity correction include at least one of crosstalk correction parameters and phasing correction parameters.

According to an embodiment of the present disclosure, the correction parameters are determined by the following steps:

identifying a plurality of high-confidence composite nucleic acid template spots among the plurality of composite nucleic acid template spots based on base-calling results;

identifying, for a given base channel, a plurality of crosstalk correction parameter reference nucleic acid template spots and a plurality of phasing correction parameter reference nucleic acid template spots among the plurality of high-confidence composite nucleic acid template spots; and

identifying, for the given base channel, the crosstalk correction parameters of the given base channel based on base-calling results of the plurality of crosstalk correction parameter reference nucleic acid template spots, and the phasing correction parameters of the given base channel based on base-calling results of the plurality of phasing correction parameter reference nucleic acid template spots, wherein the base-calling results include signal intensity values of each base channel in each of the plurality of sequencing reaction cycles.

According to an embodiment of the present disclosure, the high-confidence composite nucleic acid template spots are composite nucleic acid template spots where the base-calling results indicate only one type of base in a given sequencing reaction cycle among the plurality of sequencing reaction cycles.

According to an embodiment of the present disclosure, for a given base channel, the crosstalk correction parameter reference nucleic acid template spots are composite nucleic acid template spots that meet the following condition: in the given sequencing reaction cycle, the base-calling results of the composite nucleic acid template spots indicate only one type of base that is different from the given base.

According to an embodiment of the present disclosure, for a given base channel, the phasing correction parameter reference nucleic acid template spots are composite nucleic acid template spots that meet the following conditions:

(a) in the given sequencing reaction cycle, the base-calling results of the composite nucleic acid template spots indicate only one type of base that is different from the given base; and
(b) in at least one of the previous or subsequent cycles of the given sequencing reaction cycle, the base-calling results of the composite nucleic acid template spots indicate only one type of the given base.

According to an embodiment of the present disclosure, in condition (b), if in the previous cycle of the given sequencing reaction cycle, the base-calling results of the composite nucleic acid template spots indicate only one type of the given base, then the phasing correction parameter reference nucleic acid templates spot is identified as a lagging phasing correction parameter reference nucleic acid templates spot.

Alternatively,
in condition (b), if in the subsequent cycle of the given sequencing reaction cycle, the base-calling results of the composite nucleic acid template spots indicate only one type of the given base, then the phasing correction parameter reference nucleic acid templates spot is identified as a leading phasing correction parameter reference nucleic acid templates spot.

According to an embodiment of the present disclosure, the crosstalk correction parameters are obtained by training the following formula with the signal intensity values of each base channel from the crosstalk correction parameter reference nucleic acid template spots:

$$yi_{(B1,N)} = \beta 0 + \beta 1 * Xi_{(B2,N)} + \beta 2 * Xi_{(B3,N)} + \beta 3 * Xi_{(B4,N)} + \epsilon,$$

where:

B1, B2, B3, and B4 represent one of base A channel, base T channel, base G channel, and base C channel, respectively, with B1 representing the given base channel;
N represents the number of the given cycle;
$yi_{(B1,N)}$ represents the signal intensity value of the given base channel in the given cycle N;
$Xi_{(B2,N)}$, $Xi_{(B3,N)}$, and $Xi_{(B4,N)}$ represent the signal intensity values of given base channels B2, B3, and B4 in the given cycle N, respectively,
$\beta 0$, $\beta 1$, $\beta 2$, and $\beta 3$ represent the crosstalk correction parameters for the given base channel, and $\varepsilon$ represents an error parameter.

According to an embodiment of the present disclosure, the phasing correction parameters further include at least one of a lagging phasing correction parameter and a leading phasing correction parameter. The phasing correction parameters are obtained by training the following formula with the signal intensity values of each base channel from the phasing correction parameter reference nucleic acid template spots:

$$yi_{(B1,M)} = \beta 01 + \beta 4 * Xi_{(B1,M-1)},$$

or

$$yi_{(B1,M)} = \beta 02 + \beta 5 * Xi_{(B1,M+1)},$$

wherein: B1 represents the given base channel, M represents the number of the given cycle, M+1 represents the number of the subsequent cycle of the given cycle, M-1 represents the number of the previous cycle of the given cycle, $\beta 01$ and $\beta 4$ represent the lagging phasing correction parameters for the given base channel, and

β02 and β5 represent the leading phasing correction parameters for the given base channel.

According to an embodiment of the present disclosure, the formula is trained with an MLR model.

In a second aspect of the present disclosure, a sequencing system is provided. According to an embodiment of the present disclosure, the system includes:

a chip, provided with a plurality of composite nucleic acid template spots, wherein a plurality of nucleic acid templates are arranged in the plurality of composite nucleic acid template spots;

a detection device, configured to hybridize the plurality of nucleic acid templates with corresponding sequencing primers thereof and to synchronously perform, by use of the sequencing primers, a plurality of sequencing reaction cycles on each of the plurality of nucleic acid templates hybridized with the sequencing primers, wherein in each of the plurality of sequencing reaction cycles, signal intensities generated by the plurality of nucleic acid templates exhibit variations from one another; and

an analysis device, configured to classify sequencing channel signals into the plurality of nucleic acid templates based on the variations in the signal intensities for each of the plurality of sequencing reaction cycles.

According to an embodiment of the present disclosure, the above sequencing system may further include at least one of the following additional technical features.

According to an embodiment of the present disclosure, the plurality of nucleic acid templates on the chip are located at different positions in the same nucleic acid molecule.

According to an embodiment of the present disclosure, the plurality of nucleic acid templates on the chip are located in different nucleic acid molecules.

According to an embodiment of the present disclosure, the plurality of nucleic acid templates on the chip are located at different positions on the same single-strand DNA molecule.

According to an embodiment of the present disclosure, the plurality of nucleic acid templates on the chip are located on different strands within a complex of multiple single-stranded DNA molecule.

According to an embodiment of the present disclosure, the plurality of nucleic acid templates include a DNA molecule composite obtained by:

1) forming a single-stranded DNA molecule by performing rolling circle amplification to extend a rolling circle amplification primer, and

2) performing multiple displacement amplification on the single-stranded DNA molecule to extend a multiple displacement amplification primer.

According to an embodiment of the present disclosure, the plurality of nucleic acid templates include a DNA cluster obtained by PCR amplification.

According to an embodiment of the present disclosure, the variations in the signal intensities generated by the plurality of nucleic acid templates in the sequencing device follow a predetermined relationship.

According to an embodiment of the present disclosure, the predetermined relationship is determined by controlling copy number variations of the plurality of nucleic acid templates or controlling concentration variations of the sequencing primers for the plurality of nucleic acid templates.

According to an embodiment of the present disclosure, controlling the copy number variations of the plurality of nucleic acid templates is achieved by controlling concentration variations of the primers for different templates or duration of polymerization extension reaction during sequencing library construction.

According to an embodiment of the present disclosure, the variations in the signal intensities generated by the plurality of nucleic acid templates are at least twofold.

According to an embodiment of the present disclosure, the rolling circle amplification primer is immobilized on the chip or is free in a solution.

According to an embodiment of the present disclosure, the multiple displacement amplification primer is immobilized on the chip or is free in a solution.

According to an embodiment of the present disclosure, the rolling circle amplification and the multiple displacement amplification are performed simultaneously on the chip.

According to an embodiment of the present disclosure, the rolling circle amplification reaction is performed on the chip prior to the multiple displacement reaction through hybridization of the multiple displacement primer.

According to an embodiment of the present disclosure, after completing the simultaneous sequencing signal acquisition, the sequencing strand may be eluted to restore the sequencing template to a single-stranded state for repeated sequencing reactions.

According to an embodiment of the present disclosure, stepwise sequencing may also be performed after obtaining a sequencing chip with composite nucleic acid template spots. In such cases, either the second-strand sequencing may be performed before the first-strand sequencing, or the first-strand sequencing may be performed before the second-strand sequencing.

According to an embodiment of the present disclosure, the analysis device further includes an intensity correction module, configured to perform signal intensity correction on the signals generated in each sequencing channel before classifying the signals into the plurality of nucleic acid templates for each sequencing cycle.

According to an embodiment of the present disclosure, the analysis device further include at least one of a crosstalk correction parameter acquisition module and a phasing correction parameter acquisition module, wherein

the crosstalk correction parameter acquisition module is configured to identify, for the given base channel, the crosstalk correction parameters of the given base channel based on base-calling results of each base channel in each of the plurality of sequencing reaction cycles of a plurality of crosstalk correction parameter reference nucleic acid template spots,

the phasing correction parameter acquisition module is configured to identify, for the given base channel, the phasing correction parameters of the given base channel based on base-calling results of each base channel in each of the plurality of sequencing reaction cycles of a plurality of phasing correction parameter reference nucleic acid template spots,

wherein the base-calling results include signal intensity values of each base channel in each of the plurality of sequencing reaction cycles.

According to an embodiment of the present disclosure, the analysis device may further include a high-confidence composite nucleic acid template spot identification module, configured to identify a plurality of high-confidence composite nucleic acid template spots among the plurality of composite nucleic acid template spots based on base-calling results.

According to an embodiment of the present disclosure, the analysis device may further include a crosstalk correction parameter reference nucleic acid template spots identification module, configured to identify, for a given base channel, a plurality of crosstalk correction parameter reference nucleic acid template spots among the plurality of high-confidence composite nucleic acid template spots.

According to an embodiment of the present disclosure, the high-confidence composite nucleic acid template spots are composite nucleic acid template spots where the base-calling results indicate only one type of base in a given sequencing reaction cycle among the plurality of sequencing reaction cycles.

According to an embodiment of the present disclosure, for a given base channel, the crosstalk correction parameter reference nucleic acid template spots are composite nucleic acid template spots that meet the following condition: in the given sequencing reaction cycle, the base-calling results of the composite nucleic acid template spots indicate only one type of base that is different from the given base.

According to an embodiment of the present disclosure, the analysis device may further include a phasing correction parameter reference nucleic acid template spots identification module, configured to identify, for a given base channel, a plurality of phasing correction parameter reference nucleic acid template spots among the plurality of high-confidence composite nucleic acid template spots.

In a third aspect of the present disclosure, a computer device is provided. According to an embodiment of the present disclosure, the computer device includes: a memory having a program stored thereon; a controller configured to execute the program stored on the memory to control sequencing reaction; and a processor configured to execute the program stored on the memory to implement the sequencing method described in the first aspect.

In a fourth aspect of the present disclosure, a computer-readable storage medium is provided. According to an embodiment of the present disclosure, the storage medium stores a program being executable by a processor to implement the sequencing method described in the first aspect.

It is to be understood that within the scope of the present disclosure, each of the above technical features of the present disclosure and each of the technical features described in detail below (e.g., in the Examples) can be combined with each other to form new or preferred technical solutions. Due to the limited space, they will not be elaborated herein.

## BRIEF DESCRIPTION OF THE DRAWINGS

FIG. 1 shows a schematic flow diagram of the multiple nucleic acid templates synchronous sequencing method according to an embodiment of the present disclosure.
FIG. 2 shows a schematic flow diagram of the multiple nucleic acid templates synchronous sequencing method according to another embodiment of the present disclosure.
FIG. 3 shows a schematic flow diagram of the multiple nucleic acid templates synchronous sequencing method according to yet another embodiment of the present disclosure.

FIG. 4 shows a schematic flow diagram of the multiple nucleic acid templates synchronous sequencing method according to yet another embodiment of the present disclosure.

FIG. 5 shows a structural apparatus diagram of the multiple nucleic acid template synchronous sequencing system according to an embodiment of the present disclosure.

FIG. 6 shows another structural apparatus diagram of the multiple nucleic acid templates synchronous sequencing system according to an embodiment of the present disclosure.

FIG. 7 shows yet another structural apparatus diagram of the multiple nucleic acid templates synchronous sequencing system according to an embodiment of the present disclosure.

FIG. 8 shows yet another structural apparatus diagram of the multiple nucleic acid templates synchronous sequencing system according to an embodiment of the present disclosure.

FIG. 9 shows the results of Sequencing Experimental Scheme I in which the first strands and the second strands are sequenced simultaneously according to an embodiment of the present disclosure.

FIG. 10 shows the results of Sequencing Experimental Scheme I in which the second strand is sequenced first, followed by the first strand, according to an embodiment of the present disclosure.

FIG. 11 shows the results of Sequencing Experimental Scheme II in which the first strands and the second strands are sequenced simultaneously according to an embodiment of the present disclosure.

FIG. 12 shows the variation in raw intensity with cycle number during simultaneous sequencing of the first strands and the second strands in Sequencing Experimental Scheme III according to an embodiment of the present disclosure.

FIG. 13A shows Sequencing Experimental Scheme IV utilizing bridge amplification to achieve signal variation by controlling the copy number of nucleic acid templates according to an embodiment of the present disclosure.

FIG. 13B shows Sequencing Experimental Scheme IV utilizing bridge amplification to achieve signal variation by controlling the concentration of sequencing primers according to an embodiment of the present disclosure.

FIG. 14 shows the results of Sequencing Experimental IV in which the first strands and the second strands are sequenced simultaneously and signal variation is achieved through primer dilution according to an embodiment of the present disclosure.

## DETAILED DESCRIPTION

The embodiments of the present disclosure are described in detail below, examples of which are illustrated in the accompanying drawings. The embodiments described below with reference to the accompanying drawings are exemplary, and intended to explain the present disclosure, rather than to limit the present disclosure.

Moreover, the terms "first" and "second" are merely intended for the purpose of description but should not be construed as an indication or implication of relative importance or an implicit indication of the number of the indicated technical features. Thus, features defined by "first" or "second" may explicitly or implicitly include at least one such feature. In the description of the present disclosure, "a plurality of" means at least two, such as two or three, unless otherwise clearly and specifically limited.

The endpoints and any values of the ranges disclosed herein are not limited to those precise ranges or values, and these ranges or values should be understood to include values approximating these ranges or values. For numerical ranges, the endpoint values of each range, the endpoint values of each range and individual point values, as well as individual point values may be combined with each other to produce one or more new numerical ranges, and such numerical ranges are considered to be specifically disclosed herein.

The term "paired-end sequencing" refers to sequencing both the 5' and 3' ends of the same DNA molecule, respectively.

### Synchronous Sequencing Method

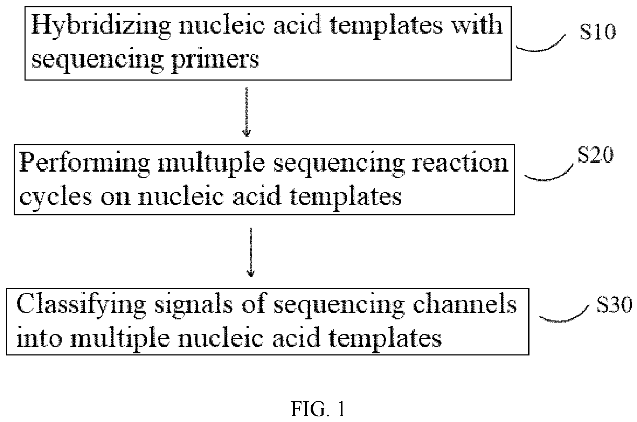

In an aspect of the present disclosure, a sequencing method is provided. In some embodiments, with reference to FIG. 1, the method includes:

S10: Hybridizing Nucleic acid templates with Sequencing Primers

During a nucleic acid sequencing process, a plurality of composite nucleic acid template spots coexist. A plurality of nucleic acid templates are arranged in the plurality of composite nucleic acid template spots. Before performing a plurality of sequencing reaction cycles on each of the plurality of nucleic acid templates, the plurality of nucleic acid templates are hybridized with their corresponding sequencing primers. In the present disclosure, the number of nucleic acid templates for synchronous sequencing is not strictly limited. Generally, at least two types of templates are required for a single composite sample spot, for example: a nucleic acid to be detected, and a barcode sequence which serves as a marker for identification. Two barcode sequences mean that three types of templates are existed.

Furthermore, in the present disclosure, the positions of the plurality of nucleic acid templates are also not strictly limited. They can be located at different positions in the same nucleic acid molecule, in different nucleic acid molecules, at different positions in the same single-stranded DNA molecule (e.g., a DNA nanoball or a linear DNA single-stranded molecule), or in different strands within a complex of multiple single-stranded DNA molecules, which can be flexibly selected according to actual situations.

As used in the present disclosure, the term "composite template" or "composite nucleic acid template" refers to include at least one sequencing template obtained by amplification in advance, with no strict limitations on the amplification method, which can be rolling circle amplification, bridge amplification, etc.. For example, the composite template may be a single-stranded DNA molecule (e.g., a DNA nanoball) formed by extending a rolling circle amplification primer using rolling circle amplification, a DNA molecule obtained by extending a multiple displacement amplification primer using multiple displacement amplification on the single-stranded DNA molecule, or a DNA cluster obtained by PCR amplification on the surface of a chip.

According to some embodiments of the present disclosure, the rolling circle amplification primer is immobilized on a solid support or the chip.

According to some embodiments of the present disclosure, the rolling circle amplification primer is free in a solution on the surface of the chip.

According to some embodiments of the present disclosure, the multiple displacement amplification primer is immobilized on a solid support or the chip.

According to some embodiments of the present disclosure, the multiple displacement amplification primer is free in a solution on the surface of the chip.

According to some embodiments of the present disclosure, the rolling circle amplification and the multiple displacement amplification are performed simultaneously in the same reaction system.

According to some embodiments of the present disclosure, the rolling circle amplification reaction is performed prior to the multiple displacement reaction through hybridization of the multiple displacement primer.

According to some embodiments of the present disclosure, the signal intensities generated by the plurality of nucleic acid templates in each of the plurality of composite nucleic acid template spots exhibit variations from one another. The sequencing channel signals are classified among the plurality of nucleic acid templates based on the variations in the signal intensities for each of the plurality of sequencing reaction cycles, to determine nucleotide sequences of the plurality of nucleic acid templates in each of the plurality of composite nucleic acid template spots.

S20: Performing a Plurality of Sequencing Reaction Cycles on Nucleic Acid Templates

In this step, after hybridizing the plurality of nucleic acid templates with their corresponding sequencing primers, a plurality of sequencing reaction cycles are performed on the plurality of nucleic acid templates by use of the sequencing primers. In each of the plurality of sequencing reaction cycles, the signal intensities generated by the plurality of nucleic acid templates exhibit variations from one another.

According to some embodiments of the present disclosure, the variations in the signal intensities generated by the plurality of nucleic acid templates follow a predetermined relationship. By controlling the number of signals generated by different nucleic acid templates, the inventors of the present application can distinguish and classify the sequenced bases into the first strand and the second strand.

In some sequencing processes of the present disclosure, the variations in the signal intensities are determined by the following methods: controlling the copy number variations of the plurality of nucleic acid templates or controlling the concentration variations of the sequencing primers for the plurality of nucleic acid templates, so as to control the relationship of the signals generated by different nucleic acid templates. Specifically, the copy number variations of the nucleic acid templates can be controlled by controlling the duration of polymerization extension reaction during sequencing library construction or by controlling the concentration variations of the primers for different nucleic acid templates. For bridge amplification, the signals generated by different nucleic acid templates can be distinguished using the following two methods. Method (1): Modifying the amplification primers on the surface of the chip. One type of amplification primer does not contain a cleavable group, and the other type of primer is mixed with a certain proportion of primers with cleavable groups (e.g., enzyme-cleavable or photo-cleavable). After amplification, removing sequencing template copies containing the cleavable group. Thus, the control of the signal relationship between the two DNA strands can be achieved. Method (2): During the sequencing process, controlling the concentration ratio of two sequencing primers, or controling the number of primers available for polymerase extension reaction by adding a blocking group at the 3' end of the sequencing primers. Specifically, the amplification primer is blocked at the 3' end by the blocking group so that it cannot be extended by polymerase. In one embodiment of the present disclosure, the blocked amplification primer is a 3' phosphorylated primer. In the presence of this blocking primer, no synthesis of nucleotide strands can occur. The blocked amplification primer can be extended by polymerase after dephosphorylation. Thus, in each of the plurality of sequencing reaction cycles, based on the principle of synchronous sequencing, each composite sample site emits fluorescence signals from two types of nucleotide sources, with variations in signal intensity, enabling better signal recognition, thereby improving the sequencing quality of synchronous sequencing.

According to some embodiments of the present disclosure, the variations in the signal intensities generated by the plurality of nucleic acid templates are at least two-fold. Those skilled in the art can set an appropriate multiplier of the the variations in signal intensity as needed, which can be 2-fold, 3-fold, 4-fold, 5-fold, 7-fold, 9-fold, 11-fold, 15-fold, 20-fold, 25-fold, 30-fold, etc.

S30: Classifying Signals of Each Sequencing Channel Into the Plurality of Templates

In this step, the sequencing channel signals are classified among the plurality of nucleic acid templates based on the variations in the signal intensities for each of the plurality of sequencing reaction cycles.

According to some embodiments of the present disclosure, the inventors have discovered that crosstalk will occur among the plurality of channels during the sequencing process, such as optical signal crosstalk between base A channel and base T channel, or optical signal crosstalk between base C channel and base G channel, resulting in inaccurate detection results of each base channel. This deviation on synchronous sequencing can be particularly significant, which results in an inaccurate distinction between two bases, thereby possibly rendering the sequencing results unusable. As a result, it is necessary to perform crosstalk correction on the signal intensities of each base channel in the image obtained from the sequencing reaction. The plurality of channels may be four channels, three channels, or two channels.

In addition, the inventors have also found that the previous or subsequent sequencing cycle will cause lagging or leading signal interference for the current sequencing cycle. As a result, it is also necessary to perform phasing correction on the sequencing signals obtained from the current sequencing cycle using the signals from the previous or subsequent sequencing cycle. Thus, correcting the signal data of each base channel achieves the purpose of improving data authenticity and removing noise and can obtain the calling information of the corrected base combination in synchronous sequencing.

Thus, for each of the plurality of sequencing reaction cycles, the signals generated by each channel are classified among the plurality of nucleic acid templates after intensity correction. The correction parameters used for intensity correction include at least one of crosstalk correction parameters and phasing correction parameters, in order to remove noise and enable base-calling in synchronous sequencing.

According to some embodiments of the present disclosure, the correction parameters are determined by: identifying a plurality of high-confidence composite nucleic acid template spots among the plurality of composite nucleic acid template spots based on base-calling results; identifying, for a given base channel, a plurality of crosstalk correction parameter reference sample spots and a plurality of phasing correction parameter reference sample spots among the plurality of high-confidence composite nucleic acid template spots; and identifying, for the given base channel, the crosstalk correction parameters of the given base channel based on base-calling results of the plurality of crosstalk correction parameter reference sample spots, and the phasing correction parameters of the given base channel based on base-calling results of the plurality of phasing correction parameter reference sample spots, wherein the base-calling results include signal intensity values of each base channel in each of the plurality of sequencing reaction cycles.

According to some embodiments of the present disclosure, the high-confidence composite nucleic acid template spots are composite nucleic acid template spots where the base-calling results indicate only one type of base in a given sequencing reaction cycle among the plurality of sequencing reaction cycles.

As used herein, "high-confidence composite sample" refers to a set of a plurality of high-confidence samples, which are primarily samples that are less interfered by signals. Because of the significant optical crosstalk between A and T bases and between C and G bases, sample spots where the base-calling results indicate only one type of base can be considered as high-confidence sample spots in a given sequencing reaction cycle, such as AA, TT, GG, and CC. Thus, the accuracy of identifying correction parameters can be ensured.

According to some embodiments of the present disclosure, for a given base channel, the crosstalk correction parameter reference sample spots include composite nucleic acid template spots that meet the following condition: in the given sequencing reaction cycle, the base-calling results of the composite nucleic acid template spot indicates only one type of base that is different from the given base. This effectively prevents significant optical crosstalk between A and T bases and between C and G bases from adversely affecting the identification of the crosstalk correction parameters.

According to some embodiments of the present disclosure, the crosstalk correction parameters are obtained by training the following formula with the signal intensity values of each base channel from the crosstalk correction parameter reference sample spots:

$$yi_{(B1,N)} = \beta0 + \beta1 * Xi_{(B2,N)} + \beta2 * Xi_{(B3,N)} + \beta3 * Xi_{(B4,N)} + \epsilon,$$

where:

B1, B2, B3, and B4 represent one of base A channel, base T channel, base G channel, and base C channel, respectively, with B1 representing the given base channel;

N represents the number of the given cycle;

$yi_{(B1,N)}$ represents the signal intensity value of the given base channel in the given cycle N;

$Xi_{(B2,N)}$, $Xi_{(B3,N)}$, and $Xi_{(B4,N)}$ represent the signal intensity values of given base channels B2, B3, and B4 in the given cycle N, respectively,

$\beta0$, $\beta1$, $\beta2$, and $\beta3$ represent the crosstalk correction parameters for the given base channel, and $\epsilon$ represents an error parameter. The formula is trained with an MLR model.

To calculate the impact factor of noise in each channel, it is necessary to first select appropriate spots. Taking the C channel as an example, the signal in the non-emission state of the C channel is attributed to crosstalk from other channels as well as phasing effects from previous and subsequent cycles. Therefore, sample spots where the current cycle is identified as GG, AA, or TT are selected to calculate the crosstalk coefficient of each channel relative to C.

According to an embodiment of the present disclosure, for a given base channel, the phasing correction parameter reference sample spots are composite nucleic acid template spots that meet the following conditions: (a) in the given sequencing reaction cycle, the base-calling results of the composite nucleic acid template spots indicate only one type of base that is different from the given base; and (b) in at least one of the previous or subsequent cycles of the given sequencing reaction cycle, the base-calling results of the composite nucleic acid template spots indicate only one type of the given base. For example, AA, TT, GG, and CC in the previous or subsequent cycle can be used as phasing correction parameter reference sample spots.

According to an embodiment of the present disclosure, in condition (b), if in the previous cycle of the given sequencing reaction cycle, the base-calling results of the composite nucleic acid template spots indicate only one type of the given base, then the phasing correction parameter reference sample spot is identified as a lagging phasing correction parameter reference sample spot. Alternatively, in condition (b), if in the subsequent cycle of the given sequencing reaction cycle, the base-calling results of the composite nucleic acid template spots indicate only one type of the given base, then the phasing correction parameter reference sample spot is identified as a leading phasing correction parameter reference sample spot.

According to an embodiment of the present disclosure, the phasing correction parameters further include at least one of a lagging phasing correction parameter and a leading phasing correction parameter. The phasing correction parameters are obtained by training the following formula with the signal intensity values of each base channel from the phasing correction parameter reference sample spots:

$$yi_{(B1,M)} = \beta01 + \beta4 * Xi_{(B1,M-1)},$$

or

$$yi_{(B1,M)} = \beta02 + \beta5 * Xi_{(B1,M+1)},$$

where: B1 represents the given base channel, M represents the number of the given cycle, M+1 represents the number of the subsequent cycle of the given cycle, M-1 represents the number of the previous cycle of the given cycle, $\beta01$ and $\beta4$ represent the lagging phasing correction parameters for the given base channel, and $\beta02$ and $\beta5$ represent the leading phasing correction parameters for the given base channel.

According to some embodiments of the present disclosure, the above formula is trained with an MLR model to obtain the crosstalk correction parameters and the phasing correction parameters.

Taking the calculation of the C channel as an example, as previously described, the signal in the non-emission state of the C channel is attributed to crosstalk from other channels as well as Lagrunon (leading phasing) effects from previous and subsequent cycles. To calculate the phasing coefficient of each channel relative to the C channel, sample spots where the current cycle is identified as GG, AA, or TT are selected. The calculation of the phasing coefficients involves the signal values of the previous and subsequent cycles, while ensuring minimal interference from the current cycle. For lagging phasing, sample spots are selected where current cycle (N) is identified as AA or TT, previous cycle (N-1) is identified as CC, and subsequent cycle (N+1) is identified as AA or TT, to calculate the lagging coefficient for the C channel. Similarly, for leading phasing, sample spots are selected where current cycle (N) is pre-called as AA or TT, previous cycle (N-1) is identified as AA or TT, and subsequent cycle (N+1) is identified as CC, to calculate the Lagrunon coefficient for the C channel. The same applies to the calculation of other channels.

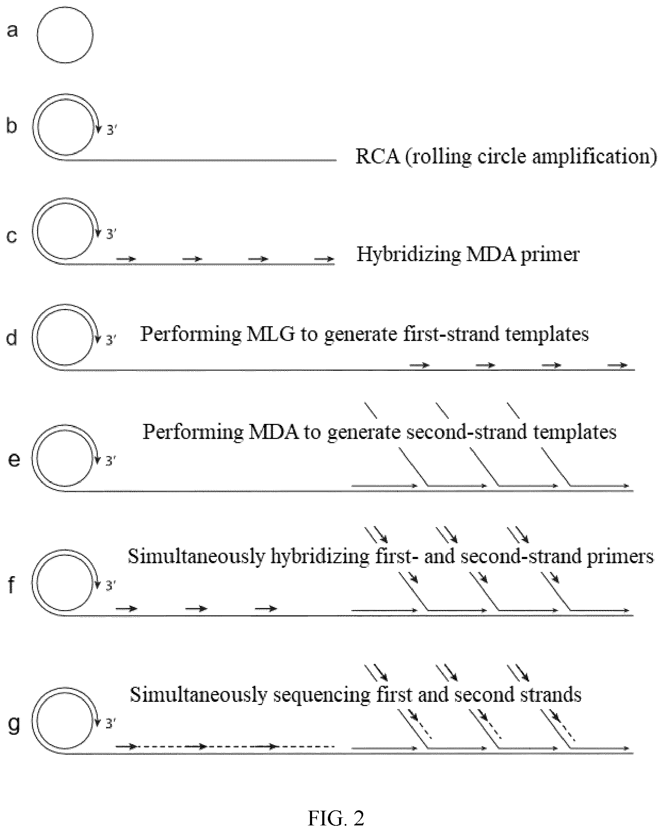

In some embodiments of the present disclosure, with reference to FIG. 2, the sequencing method includes:

Step a: Obtaining a single-stranded circular nucleic acid.

Step b: Performing rolling circle amplification (RCA) or reverse transcription reaction using the single-stranded circular nucleic acid as templates to form DNA nanoballs (DNBs).

Step c: Mixing the DNBs with a buffer solution and loading them on a sequencing slide; subsequently hybridizing multiple displacement amplification (MDA) primers.

Step d: Pumping a specific buffer solution to allow the DNBs on the chip to continue the rolling circle amplification and extend the 3' end of the template strands. The extended templates subsequently serve as the first-strand nucleic acid templates.

Step e: Performing multiple displacement amplification using the MDA primers hybridized in step c to generate the second-strand template.

At this stage, the first- and second-strand nucleic acid templates have been generated. By controlling the time of rolling circle amplification reaction, different copy numbers are obtain, resulting in variations in signal intensity between the first and the second strands using these templates.

Step f: Simultaneously hybridizing the first- and second-strand primers.

Step g: Simultaneously sequencing the first and the second strands.

The sequencing process can be repeated by eluting the hybridized first- and second- read strands to restore the single-stranded first- and second-strand templates. It can be used either for sequencing the second strand followed by the first strand or for sequencing the first strand followed by the second strand. Repeated sequencing can be achieved.

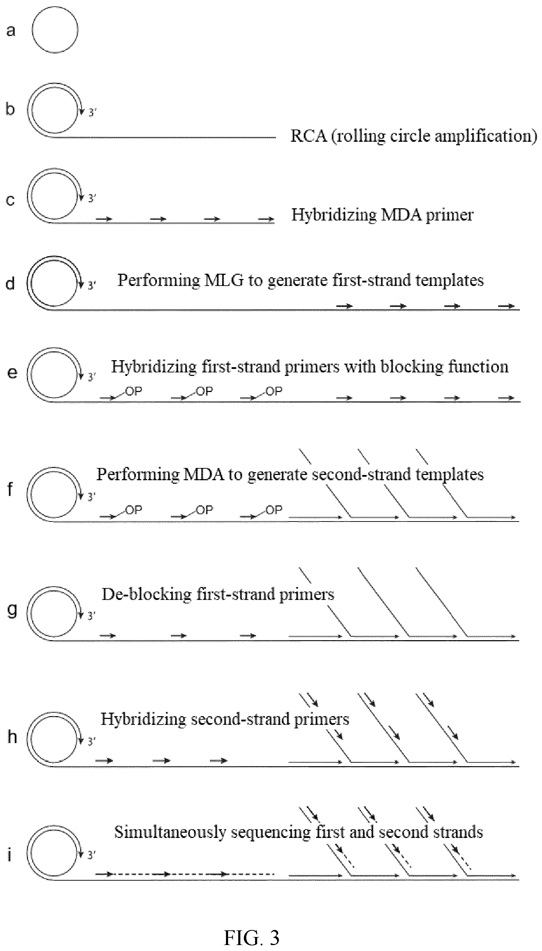

In some embodiments of the present disclosure, with reference to FIG. 3, the method includes:

Step a: Obtaining a single-stranded circular DNA.

Step b: Mixing the single-stranded circular DNA with a buffer solution, loading them on a sequencing slide, immobilizing rolling circle amplification primers on the slide, and performing rolling circle amplification (RCA) using the single-stranded circular DNA as templates to form single-stranded DNA molecules as the first-strand nucleic acid templates.

Step c: Hybridizing multiple displacement amplification (MDA) primers that are free in a solution on the surface of the slide,

Step d: Performing MLG to generate the first-strand nucleic acid templates.

Step e: Hybridizing the first-strand template with the first-strand sequencing primers with a blocking function at the 3' end.

Step f: Performing multiple displacement amplification using the MDA primers hybridized in step c to generate second-strand templates.

Step g: De-blocking the first-strand sequencing primers.

Step h: Hybridizing the second-strand primers.

Step i: Simultaneously sequencing the first and the second strands.

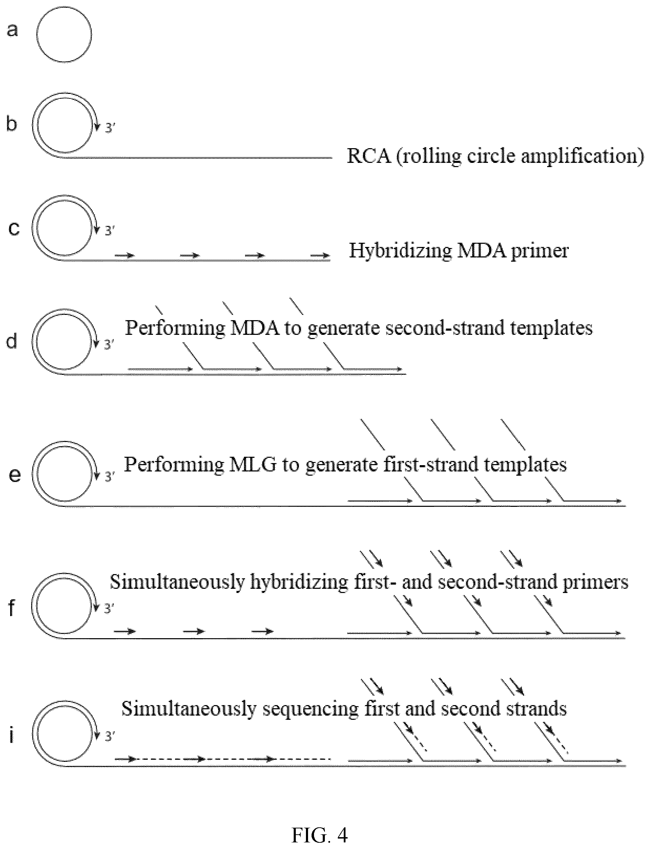

In some embodiments of the present disclosure, with reference to FIG. 4, the method includes:

Step a: Using a single-stranded circular DNA as templates.

Step b: Mixing the single-stranded circular DNA with a buffer solution, loading them on a sequencing slide, and performing rolling circle amplification (RCA) to form DNA nanoballs (DNBs).

Step c: Hybridizing multiple displacement amplification (MDA) primers that is immobilized on a sequencing slide,

Step d: Performing multiple displacement amplification using the MDA primers hybridized in step c to generate second-strand templates,

Step e: Loading a specific buffer solution to allow the DNBs on the slide to continue rolling circle amplification and extend the 3' end of the template strands. The extended templates subsequently serve as first-strand nucleic acid templates.

At this stage, the first- and second-strand nucleic acid templates have been generated. By controlling the copy number of rolling circle amplification, variations in signal intensity between the first and the second strands can be achieved using these templates.

Step f: Simultaneously hybridizing the first- and second-strand primers.

Step g: Simultaneously sequencing the first strand and the second strand.

For paired-end sequencing, it can be used either for sequencing the second strand followed by the first strand or for sequencing the first strand followed by the second strand.

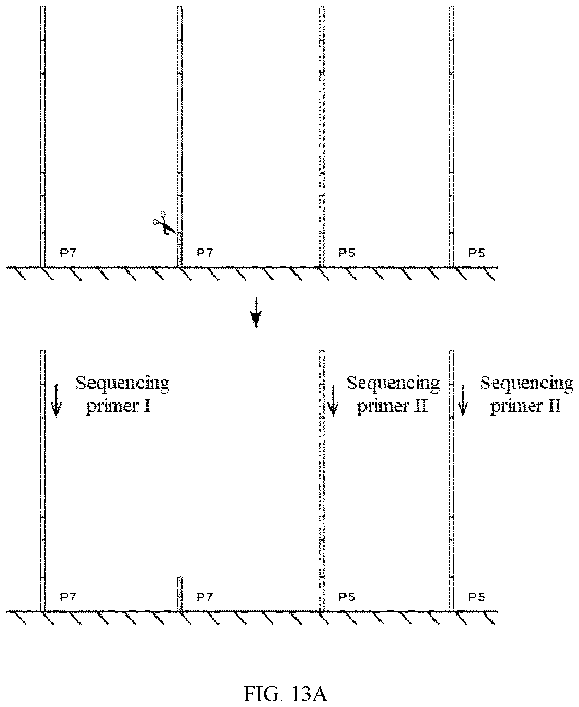

Moreover, bridge PCR amplification may also be performed to generate DNA clusters. In some embodiments of the present disclosure, with reference to FIG. 13A, the method includes:

Step a: Using a double-stranded DNA library as templates.
Step b: Performing bridge PCR amplification on a sequencing chip to generate DNA clusters.
Step c: Removing a portion of the templates from each DNA cluster by light irradiation or enzymatic cleavage reaction to form single-stranded first and second DNA strands.
Step d: Simultaneously hybridizing first- and second-strand primers, where the concentrations of the first- and second-strand primers show a multiple difference, for example, in a ratio of 1:2, 1:3, or 1: 4.
Step e: Simultaneously sequencing the first strand and the second strand.

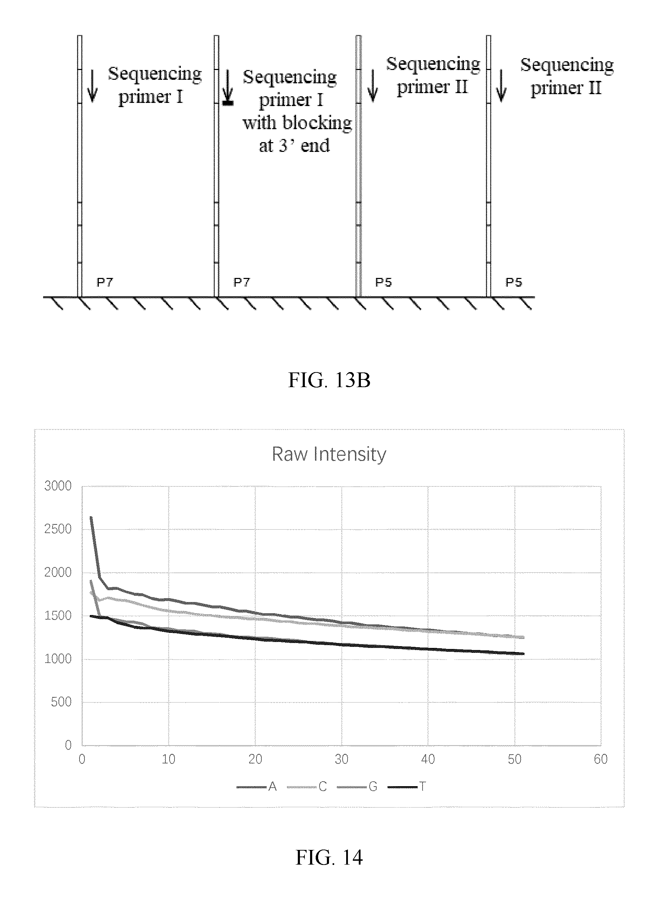

Moreover, in some embodiments of the present disclosure, with reference to FIG. 13B, the method includes:

Step a: Using a double-stranded DNA library as templates.
Step b: Performing bridge PCR amplification on a sequencing chip to generate DNA clusters.
Step c: Removing the second strand from each double-stranded DNA by light irradiation, enzymatic cleavage, or other reactions to form the same number of single-stranded first DNA strands.
Step d: Simultaneously hybridizing insert-fragment sequencing primers and barcode sequencing primers, where the concentrations of the insert-fragment sequencing primers and the barcode sequencing primers show a multiple difference, for example, in a ratio of 1:2, 1:3, or 1:4.
Step e: Simultaneous sequencing the insert-fragment strands and the barcode strands.

**Sequencing System and Apparatus**

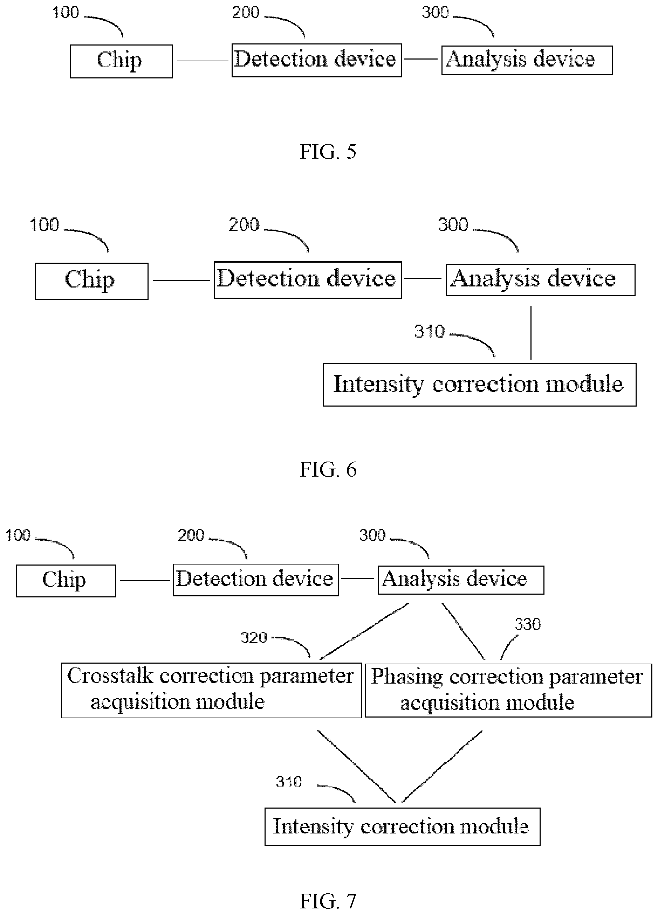

In another aspect of the present disclosure, a sequencing system is provided, as shown in FIG. 5, and includes:

a chip 100, which is a sequencing chip provided with a plurality of composite nucleic acid template spots, wherein a plurality of nucleic acid templates are arranged in the plurality of composite nucleic acid template spots;
a detection device 200, configured to hybridize the plurality of nucleic acid templates with corresponding sequencing primers and to synchronously perform, by use of the sequencing primers, a plurality of sequencing reaction cycles on each of the plurality of nucleic acid templates hybridized with the sequencing primers, wherein in each of the plurality of sequencing reaction cycles, signal intensities generated by the plurality of nucleic acid templates exhibit variations from one another; and
an analysis device 300, configured to classify sequencing channel signals among the plurality of nucleic acid templates based on the variations in the signal intensities for each of the plurality of sequencing reaction cycles.

According to some embodiments of the present disclosure, the plurality of nucleic acid templates on the chip are located at different positions in the same nucleic acid molecule, located in different nucleic acid molecules, located at different positions in the same single-stranded DNA molecule (e.g., a DNA nanoball), or in different strands within a complex of multiple single-stranded DNA molecules, which can be flexibly selected according to actual situations.

As described above, the plurality of nucleic acid templates may be a DNA molecule composite obtained by forming a single-stranded DNA molecule by extending a rolling circle amplification primer using rolling circle amplification and extending a multiple displacement amplification primer using multiple displacement amplification on the single-stranded DNA molecule, or a DNA cluster obtained by PCR amplification. The rolling circle amplification primer is immobilized on a solid support or is free in a solution. The multiple displacement amplification primer is immobilized on a solid support or is free in a solution. The rolling circle amplification and the multiple displacement amplification can be performed simultaneously in the same reaction system, or the rolling circle amplification reaction is performed prior to the multiple displacement reaction through hybridization of the multiple displacement primer.

According to some embodiments of the present disclosure, the signal intensities generated by the plurality of nucleic acid templates in each of the plurality of composite nucleic acid template spots exhibit variations from one another. The sequencing channel signals are classified among the plurality of nucleic acid templates based on the variations in the signal intensities for each of the plurality of sequencing reaction cycles, to determine nucleotide sequences of the plurality of nucleic acid templates in each of the plurality of composite nucleic acid template spots.

According to some embodiments of the present disclosure, the variations in the signal intensities generated by the plurality of nucleic acid templates in the sequencing device follow a predetermined relationship.

According to some embodiments of the present disclosure, the relationship of the signals generated by the plurality of nucleic acid templates is set by controlling the copy number variations of the plurality of templates or controlling the concentration variations of the sequencing primers for the plurality of templates.

According to some embodiments of the present disclosure, the predetermined relationship is determined by controlling copy number variations of the plurality of nucleic acid templates or controlling concentration variations of the sequencing primers for the plurality of nucleic acid templates.

According to some embodiments of the present disclosure, controlling the copy number variations of the plurality of nucleic acid templates is achieved by controlling the duration of polymerization extension reaction during sequencing library construction.

According to some embodiments of the present disclosure, the variations in the signal intensities generated by the plurality of nucleic acid templates are at least twofold.

With reference to FIG. 6, according to an embodiment of the present disclosure, the analysis device includes an intensity correction module 310, configured to perform intensity correction on the signals generated in each sequencing channel before classifying the signals among the plurality of nucleic acid templates for each sequencing cycle.

With reference to FIG. 7, according to some embodiments of the present disclosure, the analysis device further includes at least one of a crosstalk correction parameter acquisition module 320 and a phasing correction parameter acquisition module 330.

The crosstalk correction parameter acquisition module 320 is configured to identify, for the given base channel, the crosstalk correction parameters of the given base channel based on base-calling results of each base channel in each of the plurality of sequencing reaction cycles of a plurality of crosstalk correction parameter reference sample spots.

The phasing correction parameter acquisition module 330 is configured to identify, for the given base channel, the phasing correction parameters of the given base channel based on base-calling results of each base channel in each of the plurality of sequencing reaction cycles of a plurality of phasing correction parameter reference sample spots.

The base-calling results include signal intensity values of each base channel in each of the plurality of sequencing reaction cycles.

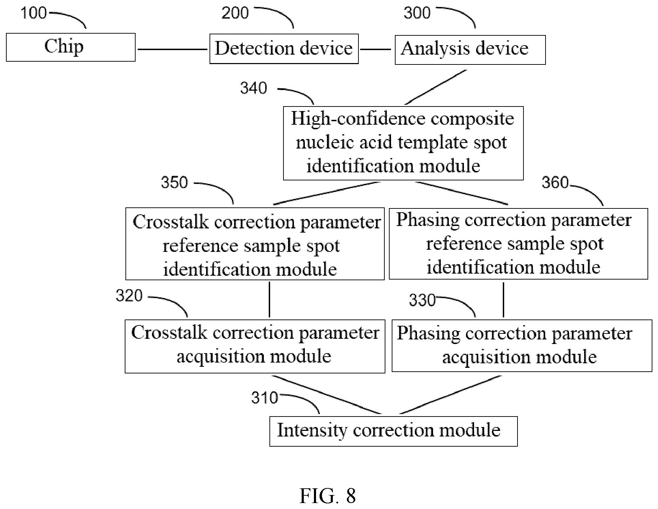

With reference to FIG. 8, according to an embodiment of the present disclosure, the analysis device may further include a high-confidence composite nucleic acid template spot identification module 340, configured to identify a plurality of high-confidence composite nucleic acid template spots among the plurality of composite nucleic acid template spots based on base-calling results. The high-confidence composite nucleic acid template spots are composite nucleic acid template spots wherein the base-calling results indicate only one type of base in a given sequencing reaction cycle among the plurality of sequencing reaction cycles.

A crosstalk correction parameter reference sample spot identification module 350, configured to identify, for a given base channel, a plurality of crosstalk correction parameter reference sample spots among the plurality of high-confidence composite nucleic acid template spots. According to some embodiments of the present disclosure, for a given base channel, the crosstalk correction parameter reference sample spots are composite nucleic acid template spots that meet the following condition: in the given sequencing reaction cycle, the base-calling results of the composite nucleic acid template spots indicate only one type of base that is different from the given base.

A phasing correction parameter reference sample spot identification module 360, configured to identify, for a given base channel, a plurality of phasing correction parameter reference sample spots among the plurality of high-confidence composite nucleic acid template spots. According to some embodiments of the present disclosure, for a given base channel, the phasing correction parameter reference sample spots are composite nucleic acid template spots that meet the following conditions: (a) in the given sequencing reaction cycle, the base-calling results of the composite nucleic acid template spots indicate only one type of base that is different from the given base; and (b) in at least one of the previous or subsequent cycles of the given sequencing reaction cycle, the base-calling results of the composite nucleic acid template spots indicate only one type of the given base. In condition (b), if in the previous cycle of the given sequencing reaction cycle, the base-calling results of the composite nucleic acid template spots indicate only one type of the given base, then the phasing correction parameter reference sample spot is identified as a lagging phasing correction parameter reference sample spot. Alternatively, in condition (b), if in the subsequent cycle of the given sequencing reaction cycle, the base-calling results of the composite nucleic acid template spots indicate only one type of the given base, then the phasing correction parameter reference sample spot is identified as a leading phasing correction parameter reference sample spot.

According to some embodiments of the present disclosure, the crosstalk correction parameter acquisition module 320 is obtained by training the following formula with the signal intensity values of each base channel from the crosstalk correction parameter reference sample spots:

$$yi_{(B1,N)} = \beta0 + \beta1 * Xi_{(B2,N)} + \beta2 * Xi_{(B3,N)} + \beta3 * Xi_{(B4,N)} + \epsilon ,$$

wherein:

B1, B2, B3, and B4 represent one of base A channel, base T channel, base G channel, and base C channel, respectively, with B1 representing the given base channel;

N represents the number of the given cycle;

$yi_{(B1,N)}$ represents the signal intensity value of the given base channel in the given cycle N;

$Xi_{(B2,N)}$, $Xi_{(B3,N)}$, and $Xi_{(B4,N)}$ represent the signal intensity values of given base channels B2, B3, and B4 in the given cycle N, respectively,

$\beta0$, $\beta1$, $\beta2$, and $\beta3$ represent the crosstalk correction parameters for the given base channel, and $\epsilon$ represents an error parameter.

According to some embodiments of the present disclosure, the phasing correction parameter acquisition module 330 is obtained by training the following formula with the signal intensity values of each base channel from the phasing correction parameter reference sample spots:

$$yi_{(B1,M)} = \beta01 + \beta4 * Xi_{(B1,M-1)},$$

or

$$yi_{(B1,M)} = \beta02 + \beta5 * Xi_{(B1,M+1)},$$

wherein: B1 represents the given base channel, M represents the number of the given cycle, M+1 represents the number of the subsequent cycle of the given cycle, M-1 represents the number of the previous cycle of the given cycle, $\beta01$ and $\beta4$ represent the lagging phasing correction parameters for the given base channel, and $\beta02$ and $\beta5$ represent the leading phasing correction parameters for the given base channel.

**Computer Product**

In yet another aspect of the present disclosure, a computer device is provided. The computer device includes a memory, a controller, and a processor. The memory has a program stored thereon. The processor is configured to execute the program stored on the memory to implement the method described in the first aspect. According to some embodiments of the present disclosure, the computer device can effectively implement the multiple- nucleic acid template synchronous sequencing method, thereby enabling the synchronous sequencing of multiple- nucleic acid templates, significantly increasing sequencing throughput and reducing sequencing time and cost. Specifically, the electronic device may be any intelligent terminal including a tablet computer, a computing cluster, a sequencer, an on-board computer, etc.

The term "memory" as used in the present disclosure refers to any computer program product, device, and/or apparatus (e.g., magnetic disk, optical disk, storage device, programmable logic device (PLD)) that provides machine instructions and/or data to a programmable processor, and includes a machine-readable medium that receives the machine instructions as machine-readable signals. The memory may be implemented in various forms, including read-only memory (ROM), a static storage device, a dynamic storage device, or a random access memory (RAM). The memory may store an operating system and other application programs. When the technical solutions provided by the embodiments of the specification are implemented via software or firmware, the relevant program codes are stored on the memory and called by the processor to execute the training methods for the gene sequencing model or the gene sequencing method in the embodiments of the present application. Specifically, the memory includes the crosstalk correction parameter acquisition module and the phasing correction parameter acquisition module.

In some embodiments, the computer device may further include an input/output interface, a communication interface, and a bus. The input/output interface is configured to implement input and output of information. The communication interface is configured to communicate and interact between the device and other devices via wired connections (e.g., USB, network cable, etc.) or wireless connections (e.g., mobile network, WIFI, Bluetooth, etc.). The bus is configured to transfer information between various components (e.g., the processor, memory, input/output interface, and communication interface) of the device.

It should be noted that the features and advantages described above with respect to the synchronous sequencing method and the method for correcting the base-calling results of synchronous sequencing are equally applicable to the synchronous sequencing system, the electronic device, and the computer-readable storage medium and will not be described here in detail.

In yet another aspect of the present disclosure, a computer-readable storage medium is provided. The storage medium stores a program being executable by a processor to implement the method described in the first aspect. The computer-readable storage medium may be any accessible medium that can be utilized by a computer or data storage device integrating one or more available media, such as servers or data centers. The accessible medium may be a magnetic medium (e.g., a floppy disk, a hard disk, or a magnetic tape), an optical medium (e.g., a digital video disc (DVD), a semiconductor medium (e.g., a solid state disk (SSD)), etc..

The present disclosure will now be described with reference to specific examples, which are intended to be illustrative only and not limiting in any way. Examples, where specific technologies or conditions are not specified, are implemented in accordance with technologies or conditions described in the literature in the art or in accordance with the product manuals. All of the used reagents or instruments, whose manufacturers are not specified, are conventional commercially available products.

**Example 1**

1. Equipment:
MGISEQ-2000 sequencer, MGISEQ-2000 sequencing reagent slide (715 nm), mini DNB loading apparatus, PCR instrument, PCR 8-tube strips, pipette set, high-speed centrifuge, mini centrifuge, and vortex mixer.
2. Reagents: the main reagents used in the present application are shown in Table 1.

Table 1:

| Reagent Name | Brand |
|---|---|
| DNA Nanoball Preparation Buffer | MGI |
| PNK Enzyme (Polynucleotide Kinase) | MGI |
| T4PNK 10X Reaction Buffer | MGI |
| MGISEQ-2000RS High-Throughput Sequencing Reagent | MGI |
| DNA Nanoball Preparation Enzyme Mix I | MGI |
| DNA Nanoball Preparation Enzyme Mix II | MGI |
| LTE Buffer | MGI |
| DNA Nanoball Termination Buffer | MGI |
| DNA Nanoball Loading Buffer IV | MGI |
| 5XSSC Buffer | MGI |
| *Escherichia Coli* Standard Library | MGI |
| 10x Phi29 Buffer | BGI Research Institute |
| First-Strand Sequencing Primer with Linker IP1-X1 Powder | Sangon |
| First-Strand Sequencing Primer with Linker IP1-X2 Powder | Sangon |
| Second-Strand Sequencing Primer IP3 | MGI |
| DNB Read Buffer (Reb) | MGI |
| EDTA | - |
| Formamide | - |

3. Primer sequences:

First-strand sequencing primer with linker IP1-x1:

CGCCG ACGCA CAGGG TGCCT CGACC GCATG GCGCG GAACC ATGGT TCCGC GCCAA CTCCT TGGCT CACAG AACGA CATGG CTACG ATCCG ACTT (94nt, SEQ ID NO: 1).

First-strand sequencing primer with linker IP1-x2:

[00250] CATGC GGTCG AGGCA CCCTG TGCGT CGGCG GGCTG CATGC CGGCA TGCAG CCCAA CTCCT TGGCT CACAG AACGA CATGG CTACG ATCCG ACTT (94nt, SEQ ID NO: 2).

First-strand sequencing primer with linker and 3' blocking IP1-x1-OP:

CGCCG ACGCA CAGGG TGCCT CGACC GCATG GCGCG GAACC ATGGT TCCGC GCCAA CTCCT TGGCT CACAG AACGA CATGG CTACG ATCCG ACTT (94nt, SEQ ID NO: 1).

First-strand sequencing primer with linker and 3' blocking IP1-x2-OP:

CATGC GGTCG AGGCA CCCTG TGCGT CGGCG GGCTG CATGC CGGCA TGCAG CCCAA CTCCT TGGCT CACAG AACGA CATGG CTACG ATCCG ACTT (94nt, SEQ ID NO: 2).

4. Reagent Preparation
1) Primer Dissolution
A 1.5-milliliter centrifuge tube containing primer powder was centrifuged at the maximum speed in an Eppendorf high-speed centrifuge (5415D) for 5 minutes. The primers were dissolved in ultrapure water in accordance with the instructions on the primer label to prepare a 100M stock solution.
2) The preparation of 1M working solution of the first-strand sequencing primer with linker, IP1-x, is shown in Table 2.

Table 2:

| Reagent Name | Volume | Final Concentration |
|---|---|---|
| 100 $\mu$M First-Strand Sequencing Primer with Linker IP1-x1 Stock Solution | 50 $\mu$L | 0.5 $\mu$M |
| 100 $\mu$M First-Strand Sequencing Primer with Linker IP1-x2 Stock Solution | 50 $\mu$L | 0.5 $\mu$M |
| 10X Phi29 Buffer | 1 mL | 1X |
| Ultrapure Water | 8.9 mL | --- |
| Total | 10 mL | --- |

3) The preparation of DNB loading buffer V is shown in Table 3.

Table 3:

| Reagent Name | Volume | Final Concentration |
|---|---|---|
| DNB Loading Buffer IV | 100 $\mu$L | --- |
| EDTA(0.5 M) | 17 $\mu$L | --- |

(continued)

| Reagent Name | Volume | Final Concentration |
|---|---|---|
| Total | 117 μL | --- |

4) The preparation of PNK enzyme reagent is shown in Table 4.

Table 4:

| Reagent Name | Final Concentration |
|---|---|
| 10U T4PNK (BGI) | 0.1U |
| T4PNK 10X Reaction Buffer (pH 5.9) | 1X |

5) The preparation of the mixed working solution of first- and second-strand sequencing primers, Insert Primer Mix, is shown in Table 5.

Table 5:

| Reagent Name | Volume | Final Concentration |
|---|---|---|
| 100 μM First-Strand Sequencing Primer with Linker IP1-x1 Stock Solution | 50 μL | 0.5 μM |
| 100 μM First-Strand Sequencing Primer with Linker IP1-x2 Stock Solution | 50 μL | 0.5 μM |
| 1.0 μM Second-Strand Sequencing Primer IP3 | 9.9 mL | 1.0 μM |
| Total | 10 mL | --- |

6) The preparation of the mixed working solution of first- and second-strand barcode primers, Barcode Primer Mix, is shown in Table 6.

Table 6:

| Reagent Name | Volume | Final Concentration |
|---|---|---|
| 100 μM First-Strand Barcode Primer BP1 Stock Solution | 100 μL | 1 μM |
| 100 μM Second-Strand Barcode Primer BP2 Stock Solution | 100 μL | 1 μM |
| 5X SSC Buffer | 9.8 mL | --- |
| Total | 10 mL | --- |

7) The preparation of 1 μM first-strand sequencing primer with linker and 3' phosphorylation working solution, IP1-x-OP, is shown in Table 7.

Table 7:

| Reagent Name | Volume | Final Concentration |
|---|---|---|
| 100 μM First-Strand Sequencing Primer with Linker IP1-x1-OP Stock Solution | 50 μL | 0.5 μM |
| 100 μM First-Strand Sequencing Primer with Linker IP1-x2-OP Stock Solution | 50 μL | 0.5 μM |
| 10X Phi29 Buffer | 1 mL | 1X |
| Ultrapure Water | 8.9 mL | --- |
| Total | 10 mL | --- |

5. Sequencing Analysis Procedure

5.1 Preparation of DNB

DNA nanoballs (DNBs) were prepared using the *Escherichia coli* library by referring to the "MGISEQ-2000RS High-throughput Sequencing Reagent Kit User Manual". The volume of EII was adjusted to 1.6 μL, and the volume of the termination buffer was reduced by half by adding 10 μL of the termination buffer.

5.2 Loading DNB

An MGISEQ-2000 sequencing reagent slide (715 nm) was prepared. The DNBs were mixed uniformly with DNB loading buffer V at a volume ratio of 2:1, and then loaded onto the MGISEQ-2000 sequencing reagent slide (715 nm) using a mini DNB loading apparatus.

5.3 Sequencing Procedure

Experimental Scheme I:

A sequencing reagent kit was prepared by referring to the "MGISEQ-2000RS High-throughput Sequencing Reagent Kit User Manual". The reagent in well 13 was replaced with 1 μM IP1-xlinker (first-strand sequencing primer working solution). The reagent in well 6 was replaced with DNB loading buffer IV. The reagent in well 7 was replaced with 1X phi29 buffer. The reagent in well 11 was replaced with REB reagent. The reagent in well 3 was replaced with Insert Primer mix (mixed solution of first- and second-strand sequencing primers). The reagent in well 8 was replaced with Barcode Primer mix (mixed solution of first- and second-strand barcode primers).

In accordance with the "MGISEQ-2000RS High-throughput Sequencing Reagent Kit User Manual", the sequencing reagent kit and the chip were placed into the MGI2000-RS sequencer. The corresponding script was selected, and SE50+10 was set for sequencing. To prove the feasibility of this scheme, the mixture of first- and second-strand sequencing primers, Insert Primer Mix, was hybridized for synchronous sequencing (50bp). Subsequently, the mixture of first- and second-strand barcode primers, Barcode Primer Mix, was hybridized for barcode sequencing (10bp).

The base-calling results of synchronous sequencing require correction. The high-confidence AA, TT, CC, and GG base combinations were screened out by a pre-calling of synchronous sequencing. The crosstalk coefficients and phasing coefficients were calculated using the MLR method and then applied to the signal correction of the whole cycle. The calculation of the phasing coefficients involved the luminance values from the previous and subsequent cycles. Therefore, in MLR, impact factors include the signals of the four channels in the current cycle and the relevant signal luminance values from the previous and subsequent cycles. To calculate the impact factor of noise in each channel, it is necessary to first select appropriate spots. The crosstalk coefficients for each base in each channel were calculated using the following formula:

$$yi_{(B1,N)} = \beta 0 + \beta 1 * Xi_{(B2,N)} + \beta 2 * Xi_{(B3,N)} + \beta 3 * Xi_{(B4,N)} + \in ,$$

wherein:

B1, B2, B3, and B4 represent one of base A channel, base T channel, base G channel, and base C channel, respectively, with B1 representing the given base channel;
N represents the number of the given cycle;
$yi_{(B1,N)}$ represents the signal intensity value of the given base channel in the given cycle N;
$Xi_{(B2,N)}$, $Xi_{(B3,N)}$, and $Xi_{(B4,N)}$ represent the signal intensity values of given base channels B2, B3, and B4 in the given cycle N, respectively,
β0, β1, β2, and β3 represent the crosstalk correction parameters for the given base channel, and ε represents an error parameter.

The calculation of the phasing coefficients involved the signal values from the previous and subsequent cycles. The formula is as follows:

$$yi_{(B1,M)} = \beta 01 + \beta 4 * Xi_{(B1,M-1)} ,$$

or

$$yi_{(B1,M)} = \beta 02 + \beta 5 * Xi_{(B1,M+1)},$$

wherein: B1 represents the given base channel, M represents the number of the given cycle, M+1 represents the number of the subsequent cycle of the given cycle, M-1 represents the number of the previous cycle of the given cycle, $\beta 01$ and $\beta 4$ represent the lagging phasing correction parameters for the given base channel, and $\beta 02$ and $\beta 5$ represent the leading phasing correction parameters for the given base channel.

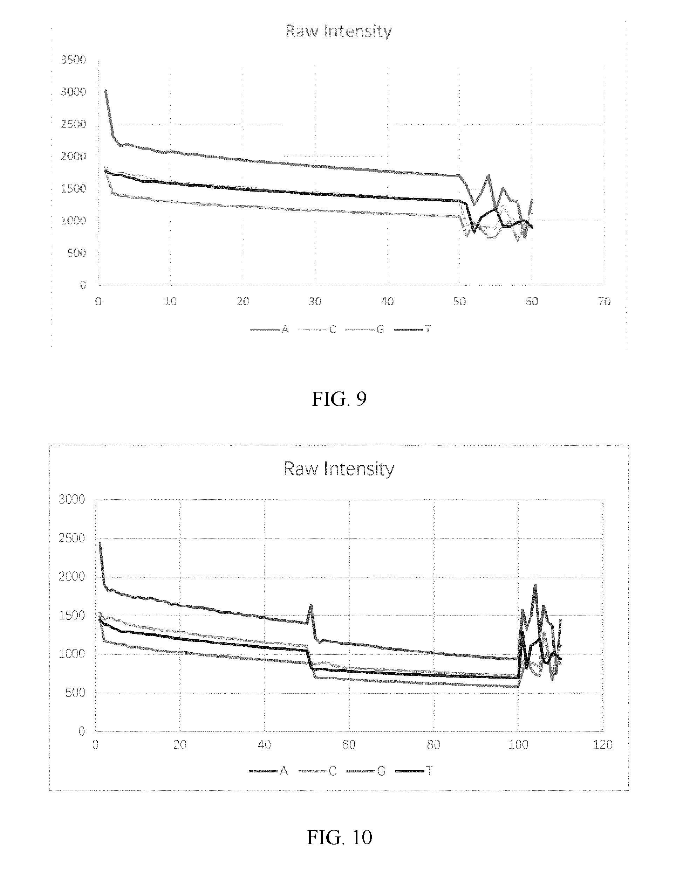

After performing the above sequencing and correction steps on a plurality of replicate wells, the final sequencing results are shown in FIG. 9 and Table 8. FIG. 9 shows the variation in raw intensity over sequencing cycles for synchronous sequencing of both first strand and the second strand across multiple replicate wells. Cycles 1-50 represent the synchronous sequencing of the first strand and the second strand. Cycles 51-60 represent the simultaneous sequencing of barcode 1 and barcode 2. The algorithm results are shown in Table 8. The synchronous results were split and aligned for analysis. For the strongest signal, the number of alignable reads was 1,139,578, with an alignment rate of 80.93% and an error rate of 0.75%. For the second strongest signal, the number of alignable reads was 213,312, with an alignment rate of 15.15% and an error rate of 3.42%. The overall error rate for synchronous sequencing in this experiment was 2%.

This scheme enables a direct assessment of the signal ratio between the first strand and the second strand templates through stepwise sequencing. FIG. 10 shows the stepwise sequencing results for the s first strand and the second strand templates under this sequencing scheme. Cycles 1-50 represent the second-strand sequencing signal results. Cycles 51-100 represent the first-strand sequencing signal results. Cycles 101-110 represent the barcode sequencing signal results.

Table 8:

| Basecall Version | Litecall |
|---|---|
| Rmax Mapping Num | 1139578 |
| Rmax Mapping Rate (%) | 80.93 |
| Rmax Error Rate (%) | 0.75 |
| Rsec Mapping Num | 213312 |
| Rsec Mapping Rate (%) | 15.15 |
| Rsec Error Rate (%) | 3.42 |
| Overall Error (%) | 2.00 |

Experimental Scheme II:

A sequencing reagent kit was prepared by referring to the "MGISEQ-2000RS High-throughput Sequencing Reagent Kit User Manual". The reagent in well 13 was replaced with 1 $\mu$M IP1-x-OP (first-strand sequencing primer with linker and 3' phosphorylation working solution ). The reagent in well 6 was replaced with DNB loading buffer IV. The reagent in well 7 was replaced with 1X phi29 buffer. The reagent in well 11 was replaced with REB reagent. The reagent in well 4 was replaced with the PNK enzyme reagent. The reagent in well 3 was replaced with Insert Primer mix (mixed solution of first- and second-strand sequencing primers). The reagent in well 8 was replaced with Barcode Primer mix (mixed solution of first- and second-strand barcode primers).

In accordance with the "MGISEQ-2000RS High-throughput Sequencing Reagent Kit User Manual", the sequencing reagent kit and the chip were placed into the MGI2000-RS sequencer. The corresponding script was selected, and SE50+10 was set for sequencing. To prove the feasibility of this scheme, the first-strand sequencing primer with phosphorylation at the 3' end was hybridized first, followed by MDA. Then, the blocking primer hybridized on the first strand was dephosphorylated, followed by hybridization with the second-strand primer for simultaneous sequencing (50bp) of the first strand and the second strand. Subsequently, the mixture of first- and second-strand barcode primers, Barcode Primer Mix, was hybridized for barcode sequencing (10bp).

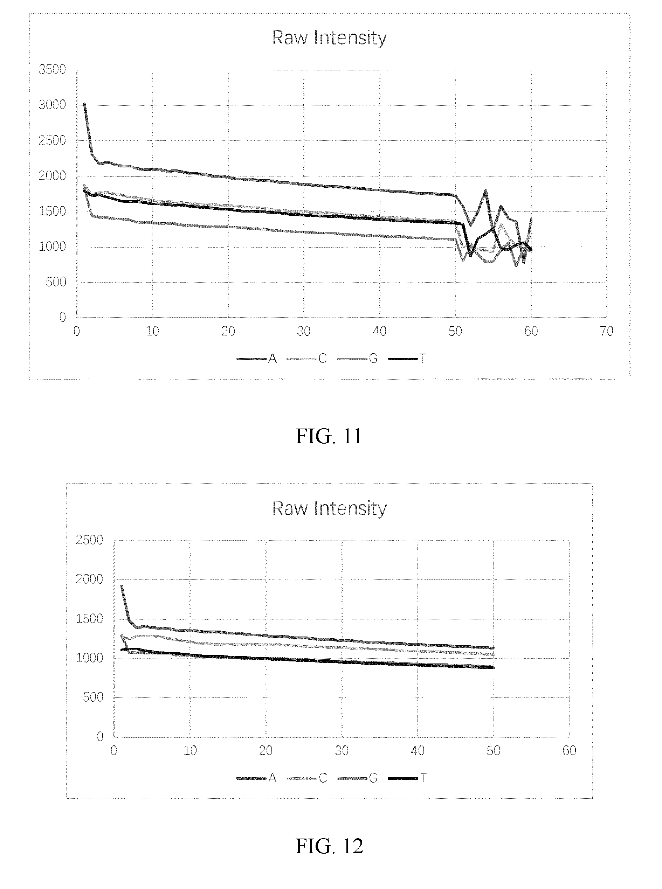

The base-calling results of synchronous sequencing were corrected in the same manner as in Experimental Scheme I. The experimental results are shown in FIG. 11. Cycles 1-50 represent the synchronous sequencing of the first strand and the second strand. Cycles 51-60 represent the simultaneous sequencing of barcode 1 and barcode 2. The algorithm results are shown in Table 9. The synchronous results were split and aligned for analysis. For the strongest signal, the number of alignable reads was 981,505, with an alignment rate of 69.71% and an error rate of 1.64%. For the second strongest signal, the number of alignable reads was 171,611, with an alignment rate of 12.18% and an error rate of 4.67%. The overall error rate for synchronous sequencing in this experiment was 3.02%.

Table 9:

| Basecall Version | Litecall |
| --- | --- |
| Rmax Mapping Num | 981505 |
| Rmax Mapping Rate (%) | 69.71 |
| Rmax Error Rate (%) | 1.64 |
| Rsec Mapping Num | 171611 |
| Rsec Mapping Rate (%) | 12.18 |
| Rsec Error Rate (%) | 4.67 |
| Overall Error (%) | 3.02 |

Experimental Scheme III:

A sequencing reagent kit was prepared by referring to the "MGISEQ-2000RS High-throughput Sequencing Reagent Kit User Manual". The reagent in well 13 was replaced with 1 μM IP1-x (first-strand sequencing primer with linker working solution). The reagent in well 6 was replaced with DNB loading buffer IV. The reagent in well 7 was replaced with 1X phi29 buffer. The reagent in well 11 was replaced with REB reagent. The reagents in wells 4 and 5 were removed. The reagent in well 3 was replaced with Insert Primer mix (mixed solution of first- and second-strand sequencing primers).
In accordance with the "MGISEQ-2000RS High-throughput Sequencing Reagent Kit User Manual", the sequencing reagent kit and the chip were placed into the MGI2000-RS sequencer. The corresponding script was selected, and SE50 was set for sequencing. The base-calling results of synchronous sequencing were corrected in the same manner as in Experimental Scheme I.
The algorithm results of synchronous sequencing are shown in Table 10. The synchronous results were split and aligned for analysis. For the strongest signal, the number of alignable reads was 937,468, with an alignment rate of 66.58% and an error rate of 1.39%. For the second strongest signal, the number of alignable reads was 500,782, with an alignment rate of 35.56% and an error rate of 2.91%. The overall error rate for synchronous sequencing in this experiment was 2.5%. FIG. 12 shows the variation in raw intensity over sequencing cycles for synchronous sequencing.

Table 10

| Basecall Version | Litecall |
| --- | --- |
| Rmax Mapping Num | 937468 |
| Rmax Mapping Rate (%) | 66.58 |
| Rmax Error Rate (%) | 1.39 |
| Rsec Mapping Num | 500782 |
| Rsec Mapping Rate (%) | 35.56 |
| Rsec Error Rate (%) | 2.91 |
| Overall Error (%) | 2.5 |

Experimental Scheme IV:

The signal variations between the first strand and second strand can also be achieved for synchronous sequencing by controlling the copy number of templates or by controlling the concentration of sequencing primers. Taking the DNA clusters generated by bridge PCR amplification as an example, the scheme achieving signal variations by controlling the copy number of nucleic acid templates is shown in FIG. 13A, and the scheme achieving signal variations by controlling the concentration of sequencing primers is shown in FIG. 13B. The 3' end of the sequencing primers was blocked by the blocking group, preventing extension by DNA polymerase. In this example, primer concentration control was taken as an example for verification. The specific experimental operations are as follows.

The first-strand sequencing primer having a blocking group at the 3' end, the conventional first-strand sequencing primer, and the second-strand sequencing primer were mixed in a ratio of 1:1:2 to obtain a sequencing primer mixture. DNA clusters were generated by bridge PCR amplification and then hybridized with the above sequencing primer mixture for synchronous sequencing.

The experimental results are shown in FIG. 14 and Table 11. FIG. 14 shows the variation in raw intensity over sequencing cycles for synchronous sequencing using the scheme achieving signal variations by controlling the concentration of sequencing primers. The base-calling results of synchronous sequencing were corrected in the same manner as in Experimental Scheme I.

The algorithm results of synchronous sequencing are shown in Table 11. The synchronous results were split and aligned for analysis. For the strongest signal, the number of alignable reads was 941,401, with an alignment rate of 66.86% and an error rate of 1.41%. For the second strongest signal, the number of alignable reads was 513,416, with an alignment rate of 36.46% and an error rate of 4.36%. The overall error rate for synchronous sequencing in this experiment was 2.92%.

Table 11: Algorithmic Results of Synchronous Sequencing via Primer Dilution for Signal Variations

| Basecall Version | Litecall |
|---|---|
| Rmax Mapping Num | 941401 |
| Rmax Mapping Rate (%) | 66.86 |
| Rmax Error Rate (%) | 1.41 |
| Rsec Mapping Num | 513416 |
| Rsec Mapping Rate (%) | 36.46 |
| Rsec Error Rate (%) | 4.36 |
| Overall Error (%) | 2.92 |

Moreover, the terms "first" and "second" are merely intended for the purpose of description but should not be construed as an indication or implication of relative importance or an implicit indication of the number of the indicated technical features. Thus, features defined by "first" or "second" may explicitly or implicitly include at least one such feature. In the description of the present disclosure, "a plurality of" means at least two, such as two or three, unless otherwise clearly and specifically limited.

In the description of the specification, reference to the term "an embodiment", "some embodiments", "an example", "a specific example" or "some examples," or the like means that a specific feature, structure, material, or characteristic described in combination with the embodiment(s) or example(s) are included in at least one embodiment or example of the present disclosure. In the specification, the schematic description of the above terms is unnecessarily against the same embodiment or example. Furthermore, the specific feature, structure, material, or characteristic described may be combined in any suitable manner in any one or more embodiments or examples. Moreover, without mutual contradiction, those skilled in the art may incorporate and combine different embodiments or examples and features of the different embodiments or examples described in the specification.

Although the embodiments of the present disclosure have been illustrated and described above, it should be understood that the above embodiments are exemplary and cannot be construed as limitations on the present disclosure. Changes, modifications, replacements, and variants to the above embodiments may be made by those skilled in the art within the scope of the present disclosure.

**Claims**

1.   A sequencing method, comprising:

   providing a plurality of composite nucleic acid template spots, with a plurality of nucleic acid templates being arranged in the plurality of composite nucleic acid template spots;
   hybridizing the plurality of nucleic acid templates with corresponding sequencing primers thereof;
   performing, by use of the sequencing primers, a plurality of sequencing reaction cycles on each of the plurality of nucleic acid templates hybridized with the sequencing primers, wherein in each of the plurality of sequencing reaction cycles, signal intensities generated by the plurality of nucleic acid templates exhibit variations from one another; and

classifying sequencing channel signals among the plurality of nucleic acid templates based on the variations in the signal intensities for each of the plurality of sequencing reaction cycles.

2. The sequencing method according to claim 1, wherein the variations in the signal intensities generated by the plurality of nucleic acid templates follow a predetermined relationship;

optionally, the predetermined relationship is determined by controlling copy number variations of the plurality of nucleic acid templates or controlling concentration variations of the sequencing primers for the plurality of nucleic acid templates;
optionally, said controlling the copy number variations of the plurality of nucleic acid templates is achieved by controlling concentration variations of the primers for different templates during sequencing library construction or duration of polymerization extension reaction during the sequencing library construction;
optionally, the variations in the signal intensities generated by the plurality of nucleic acid templates are at least twofold.

3. The sequencing method according to claim 1 or 2, wherein the plurality of nucleic acid templates are located at different positions in the same nucleic acid molecule;
optionally, the plurality of nucleic acid templates are located in different nucleic acid molecules.

4. The sequencing method according to claim 3, wherein the plurality of nucleic acid templates comprise a DNA molecule composite obtained by:

forming a single-stranded DNA molecule by performing rolling circle amplification to extend a rolling circle amplification primer; and
performing multiple displacement amplification on the single-stranded DNA molecule to extend a multiple displacement amplification primer;
optionally, the plurality of nucleic acid templates comprise a DNA cluster obtained by PCR amplification.

5. The sequencing method according to claim 4, wherein the rolling circle amplification primer is immobilized on a solid support or is free in a solution;
optionally, the multiple displacement amplification primer is immobilized on a solid support or is free in a solution.

6. The sequencing method according to claim 4, wherein the rolling circle amplification and the multiple displacement amplification are performed simultaneously in the same reaction system.

7. The sequencing method according to claim 4, wherein the rolling circle amplification reaction is performed prior to the multiple displacement reaction through hybridization of the multiple displacement primer.

8. The sequencing method according to claim 1, wherein:

the signal intensities generated by the plurality of nucleic acid templates in each of the plurality of composite nucleic acid template spots exhibit variations from one another; and
the signals of the sequencing channels are classified among the plurality of nucleic acid templates based on the variations in the signal intensities for each of the plurality of sequencing reaction cycles, to determine nucleotide sequences of the plurality of nucleic acid templates in each of the plurality of composite nucleic acid template spots.

9. A sequencing system, comprising:

a chip, provided with a plurality of composite nucleic acid template spots, wherein a plurality of nucleic acid templates are arranged in the plurality of composite nucleic acid template spots;
a detection device, configured to: hybridize the plurality of nucleic acid templates with corresponding sequencing primers thereof, and synchronously perform, by use of the sequencing primers, a plurality of sequencing reaction cycles on each of the plurality of nucleic acid templates hybridized with the sequencing primers, wherein in each of the plurality of sequencing reaction cycle, signal intensities generated by the plurality of nucleic acid templates exhibit variations from one another; and
an analysis device, configured to classify sequencing channel signals among the plurality of nucleic acid templates based on the variations in the signal intensities for each of the plurality of sequencing reaction cycles.

**10.** The sequencing system according to claim 9, wherein the plurality of nucleic acid templates on the chip are located at different positions in the same nucleic acid molecule;

optionally, the plurality of nucleic acid templates on the chip are located in different nucleic acid molecules;
optionally, the plurality of nucleic acid templates comprise a DNA molecule obtained by:

1) forming a single-stranded DNA molecule by performing rolling circle amplification to extend a rolling circle amplification primer; and
2) performing multiple displacement amplification on the single-stranded DNA molecule to extend a multiple displacement amplification primer;

optionally, the plurality of nucleic acid templates comprise a DNA cluster obtained by PCR amplification.

**11.** The sequencing system according to claim 9, wherein the variations in the signal intensities generated by the plurality of nucleic acid templates in the sequencing device follow a predetermined relationship;

optionally, the predetermined relationship is determined by controlling copy number variations of the plurality of nucleic acid templates or controlling concentration variations of the sequencing primers for the plurality of nucleic acid templates;
optionally, said controlling the copy number variations of the plurality of nucleic acid templates is achieved by controlling concentration variations of the primers for different templates during sequencing library construction or duration of polymerization extension reaction during the sequencing library construction;
optionally, the variations in the signal intensities generated by the plurality of nucleic acid templates are at least twofold.

**12.** The sequencing system according to claim 10, wherein the rolling circle amplification primer is immobilized on the chip or is free in a solution;
optionally, the multiple displacement amplification primer is immobilized on the chip or is free in a solution.

**13.** The sequencing system according to claim 10, wherein the rolling circle amplification and the multiple displacement amplification are performed simultaneously on the chip.

**14.** The sequencing system according to claim 10, wherein the rolling circle amplification reaction is performed on the chip prior to the multiple displacement reaction through hybridization of the multiple displacement primer.

**15.** The sequencing system according to claim 9, wherein:

the signal intensities generated by the plurality of nucleic acid templates in each of the pluratliy of composite nucleic acid template spots on the chip exhibit variations from one another; and
the sequencing channel signals are classified among the plurality of nucleic acid templates based on the variations in the signal intensities for each of the plurality of sequencing reaction cycles using the analysis device, to determine nucleotide sequences of the plurality of nucleic acid templates in each of the pluratliy of composite template sample spots.

**16.** A sequencing device, comprising:

a memory having a program stored thereon; and
a processor, configured to execute the program stored on the memory to implement the sequencing method according to any one of claims 1 to 8.

**17.** A computer-readable storage medium, having a program stored thereon, the program being executable by a processor to implement the sequencing method according to any one of claims 1 to 8.

Hybridizing nucleic acid templates with sequencing primers ⟍ S10

↓

Performing multuple sequencing reaction cycles on nucleic acid templates ⟍ S20

↓

Classifying signals of sequencing channels into multiple nucleic acid templates ⟍ S30

FIG. 1

FIG. 2

a

b $\quad$ 3'

RCA (rolling circle amplification)

c $\quad$ 3'

Hybridizing MDA primer

d $\quad$ 3'

Performing MLG to generate first-strand templates

e $\quad$ 3'

Hybridizing first-strand primers with blocking function

OP $\quad$ OP $\quad$ OP

f $\quad$ 3'

Performing MDA to generate second-strand templates

OP $\quad$ OP $\quad$ OP

g $\quad$ 3'

De-blocking first-strand primers

h $\quad$ 3'

Hybridizing second-strand primers

i $\quad$ 3'

Simultaneously sequencing first and second strands

FIG. 3

FIG. 4

FIG. 5

FIG. 6

FIG. 7

100 ⌐

300 ⌐

Chip ———— Detection device ——— Analysis device

340 ⌐

High-confidence composite
nucleic acid template spot
identification module

350 ⌐

360 ⌐

Crosstalk correction parameter
reference sample spot
identification module

Phasing correction parameter
reference sample spot
identification module

320 ⌐

330 ⌐

Crosstalk correction parameter
acquisition module

Phasing correction parameter
acquisition module

310 ⌐

Intensity correction module

FIG. 8

FIG. 9

FIG. 10

FIG. 11

FIG. 12

FIG. 13A

FIG. 13B

FIG. 14

## INTERNATIONAL SEARCH REPORT

| International application No. |
| --- |
| **PCT/CN2022/138468** |

| A. | CLASSIFICATION OF SUBJECT MATTER |
| --- | --- |

C12Q 1/68(2018.01)i

According to International Patent Classification (IPC) or to both national classification and IPC

| B. | FIELDS SEARCHED |
| --- | --- |

Minimum documentation searched (classification system followed by classification symbols)

    IPC：C12Q

Documentation searched other than minimum documentation to the extent that such documents are included in the fields searched

Electronic data base consulted during the international search (name of data base and, where practicable, search terms used)

    CNMED, CNABS, DWPI, WPABS, WPABSC, CJFD, AUABS, TWMED, ILABS, HKABS, MOABS, CNTXT, ENTXT, ENTXTC, WOTXT, USTXT, VCN, VEN, GBTXT, EPTXT, CATXT, CNKI, PubMed, EMBL, Web of Science, GoogleScholar: 测序, 信号, 差异, Sequenc+, PCR, 数据, data, signal, differ+, 增量, 偏差, 差别, 区别

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2022349001 A1 (MGI TECH CO., LTD.) 03 November 2022 (2022-11-03) <br> claims 1-5 | 1-17 |
| A | CN 114774523 A (APOGENOMICS CO., LTD.; NANJING JUNHUA GENE TECHNOLOGY CO., LTD.) 22 July 2022 (2022-07-22) <br> description, paragraphs 6 and 7 | 1-17 |
| A | WO 2022247555 A1 (GENEMIND BIOSCIENCES CO., LTD.) 01 December 2022 (2022-12-01) <br> description, pages 2-3 | 1-17 |
| A | US 2017298430 A1 (ILLUMINA CAMBRIDGE LIMITED) 19 October 2017 (2017-10-19) <br> abstract | 1-17 |
| A | US 2022349002 A1 (PACIFIC BIOSCIENCES OF CALIFORNIA, INC.) 03 November 2022 (2022-11-03) <br> abstract | 1-17 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **30 August 2023** | **09 September 2023** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

| International application No. |
| --- |
| **PCT/CN2022/138468** |

| Patent document cited in search report | | | Publication date (day/month/year) | Patent family member(s) | | | Publication date (day/month/year) |
| --- | --- | --- | --- | --- | --- | --- | --- |
| US | 2022349001 | A1 | 03 November 2022 | CA | 3164884 | A1 | 22 July 2021 |
| | | | | AU | 2020423361 | A1 | 15 September 2022 |
| | | | | JP | 2023510424 | A | 13 March 2023 |
| | | | | EP | 4092135 | A1 | 23 November 2022 |
| | | | | EP | 4092135 | A4 | 18 January 2023 |
| | | | | WO | 2021142769 | A1 | 22 July 2021 |
| CN | 114774523 | A | 22 July 2022 | | None | | |
| WO | 2022247555 | A1 | 01 December 2022 | | None | | |
| US | 2017298430 | A1 | 19 October 2017 | HK | 1243465 | A1 | 13 July 2018 |
| | | | | SG | 11201703629 | XA | 29 June 2017 |
| | | | | AU | 2015341568 | A1 | 25 May 2017 |
| | | | | AU | 2015341568 | B2 | 16 September 2021 |
| | | | | US | 2023160006 | A1 | 25 May 2023 |
| | | | | GB | 201419731 | D0 | 17 December 2014 |
| | | | | AU | 2021286342 | A1 | 20 January 2022 |
| | | | | WO | 2016071689 | A1 | 12 May 2016 |
| | | | | EP | 3215638 | A1 | 13 September 2017 |
| | | | | EP | 3215638 | B1 | 11 August 2021 |
| | | | | ES | 2889875 | T3 | 14 January 2022 |
| | | | | EP | 3974538 | A1 | 30 March 2022 |
| | | | | CA | 2966442 | A1 | 12 May 2016 |
| | | | | US | 2020392573 | A1 | 17 December 2020 |
| | | | | US | 11555218 | B2 | 17 January 2023 |
| | | | | DK | 3215638 | T3 | 18 October 2021 |
| | | | | JP | 2017533709 | A | 16 November 2017 |
| | | | | JP | 6789935 | B2 | 25 November 2020 |
| US | 2022349002 | A1 | 03 November 2022 | EP | 4114966 | A1 | 11 January 2023 |
| | | | | CA | 3173798 | A1 | 10 September 2021 |
| | | | | AU | 2021230282 | A1 | 22 September 2022 |
| | | | | US | 11608528 | B2 | 21 March 2023 |
| | | | | WO | 2021178467 | A1 | 10 September 2021 |

Form PCT/ISA/210 (patent family annex) (July 2022)